(19) 

Europäisches
Patentamt
European
Patent Office
Office européen
des brevets

(11) **EP 4 711 824 A1**

(12) **EUROPEAN PATENT APPLICATION**

(43) Date of publication:
**18.03.2026 Bulletin 2026/12**

(21) Application number: **25202120.9**

(22) Date of filing: **15.09.2025**

(51) International Patent Classification (IPC):
**G01T 1/29** (2006.01) **A61B 6/03** (2006.01)

(52) Cooperative Patent Classification (CPC):
**G01T 1/2985; A61B 6/037**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **14.09.2024 CN 202411298329**

(71) Applicant: **Shanghai United Imaging Healthcare
Co., Ltd.
Shanghai 201807 (CN)**

(72) Inventors:
• **REN, Jin
Shanghai 201807 (CN)**
• **LIU, Yilin
Shanghai 201807 (CN)**
• **LV, Yang
Shanghai 201807 (CN)**
• **DONG, Yun
Shanghai 201807 (CN)**

(74) Representative: **Murgitroyd & Company
165-169 Scotland Street
Glasgow G5 8PL (GB)**

(54) **METHOD FOR IDENTIFYING PROMPT-GAMMA EVENT, PET IMAGING METHOD, AND COMPUTER DEVICE**

(57)     The present disclosure relates to a method for identifying a prompt-γ event, which includes: obtaining first time-of-flight (TOF) data of multiple double coincidence events detected by a detector device; determining second TOF data of one or more triple coincidence events based on the first TOF data, performing verifica- tion operations on the second TOF data of the triple coincidence events to determine a prompt-γ event in each of the triple coincidence events. The embodiments of this application can effectively identify prompt gamma photon events by using TOF data, which is helpful for separating dual-nuclide signals.

FIG. 1

**EP 4 711 824 A1**

## Description

## TECHNICAL FIELD

The present disclosure relates to the field of medical technologies, and in particular, to a method for identifying a prompt-γ event d and a computer device.

## BACKGROUND

Positron Emission Tomography (PET), one of the most highly sensitive and specific in vivo molecular imaging methods available today, has become an important tool for tumor research and clinical diagnosis and treatment. The principle of PET imaging is that during the decay of a nuclide, positrons are emitted, which interact with electrons in the surrounding material to produce annihilation radiation, emitting two photons that are opposite in direction but equal in energy. Based on these two photons, a tomographic distribution map of the body's positron nuclide is obtained to show the location, shape, size and metabolic function of the lesion for the diagnosis of the disease.

Conventional PET single-scan imaging can only perform functional imaging of one tracer and has limited characterizing ability for complex life activities. Multiple PET scans can provide more comprehensive information about life activities, but they are costly in terms of economy and time, and registration problems between multiple scans can also affect the final imaging quality. Therefore, single PET scan imaging with multiple tracers is of great significance in clinical applications.

However, some nuclides, while decaying, emit prompt-γ rays with a certain probability, which can produce triple coincidence events after positron annihilation. However, in traditional multi-tracer single PET scan imaging, only double coincidence events can be identified and output, and prompt-γ events cannot be identified in triple coincidence events, which leads to the inability to effectively separate the double nuclide signals.

## SUMMARY

In a first aspect, the present disclosure provides a method for identifying a prompt-γ event. The method includes: obtaining first time-of-flight (TOF) data of multiple double coincidence events detected by a detector device, wherein each of the double coincidence events corresponds to a pair of photons associated with an annihilation event; determining second TOF data of one or more triple coincidence events based on the first TOF data, wherein each of the triple coincidence events corresponds to three photons any two of which are coincident; performing verification operations on the second TOF data of the triple coincidence events to determine a prompt-γ event in each of the triple coincidence events.

Each of the verification operations includes: determining estimated TOF data corresponding to each of the triple coincidence events based on detection positions and TOF information included in the second TOF data; and determining a prompt-γ event in each of the triple coincidence events by comparing the estimated TOF data with the second TOF data.

In some embodiments, the determining the estimated TOF data includes: selecting candidate true double coincidence event and a candidate prompt-γ photon based on each of the triple coincidence events; determining a candidate decay position based on the detection position and TOF information of the candidate true double coincidence event; determining an estimated TOF data of a corresponding triple coincidence event based on the candidate decay position, the detection position of the candidate true double coincidence event, and a detection position of the candidate prompt-γ photon.

In some embodiments, determining the prompt-γ event in each of the triple coincidence events by comparing the estimated TOF data with the second TOF data includes: comparing the estimated TOF data with the second TOF data, in response to the estimated TOF data matching the second TOF data, determining the candidate prompt-γ photon as the true prompt-γ photon; or, in response to the estimated TOF data not matching the second TOF data, reselecting another candidate true double coincidence event and another candidate prompt-γ photon.

In some embodiments, the estimated TOF data matching the second TOF data further includes: determining a TOF difference between the estimated TOF data and the second TOF data; confirming that the estimated TOF data matches the second TOF data if the TOF difference is less than a difference threshold.

In some embodiments, determining the TOF difference between the estimated TOF data and the second TOF data includes: determining a reference TOF vector based on the second TOF data; obtaining TOF deference by calculating the distance between the TOF vector corresponding to the estimated TOF data and the reference TOF vector.

In some embodiments, the determining a candidate decay position based on the detection position and TOF information of the candidate true double coincidence events, including: determining one or more weights based on the detection position and TOF information of the candidate true double coincidence events; obtaining candidate decay positions by calculating a weighted sum of the detection positions of the candidate true double coincidence events based on the weights.

In some embodiments, determining the second TOF data of one or more triple coincidence events based on the first TOF data, including: determining double coincidence events with the same detection position based on the first TOF data; determining the second TOF data based on the double coincidence events with the same detection position.

In some embodiments, he double coincidence events with the same detection position include three double coincidence events, and any two of the three double coincidence events have a same detection position; the determining the second TOF data based on the double coincidence events with the same detection position, including: checking whether TOF information of the three double coincidence events satisfies a time condition; generating second TOF data based on the detection position and TOF information of the three double coincidence events if the TOF information of the three double coincidence events satisfies the time condition.

In a second aspect, the present disclosure further provides a PET imaging method, including: obtaining PET scanning data; determining multiple double coincidence events corresponding to first TOF data based on the PET scanning data; determining multiple triple coincidence events based on the first TOF data, wherein the triple coincidence events correspond to second TOF data and each of the triple coincidence events corresponds to three photons any two of which are coincident; determining true double coincidence events based on detection positions and TOF information included in the second TOF data; reconstructing a PET image based on the true double coincidence events.

In a third aspect, the present disclosure further provides an electronic computer device, including a memory, a processor, the memory storing a computer program.

The processor performs the following steps when executing the computer program: obtaining PET scanning data, the PET scanning data are obtained based on a first tracer and a second tracer, and the second tracer is different from the first tracer; determining multiple double coincidence events based on the PET scanning data, with each pair of double coincidence events corresponding to a first TOF data; determining a first subset of double-coincidence events and multiple triple-coincidence events from the multiple double-coincidence events, based on the first TOF data; wherein triple-coincidence events correspond to second TOF data, and the first subset of double-coincidence events is associated with the first tracer; determining a second subset of double coincidence events from the multiple triple-coincidence events based on the second TOF data, and the second subsets of double coincidence events are associated with the second tracer; reconstructing first image related to the first tracer based on the first subset of double coincidence events and second image related to the second tracer based on the second subset of double coincidence events.

In some embodiments, the second tracer is different from the first tracer, including: one of the first tracer and the second tracer includes $\beta^+$ particle decay, and the other one includes $\beta^+\gamma$ decay.

One or more embodiments of the present disclosure will be described in detail below with reference to drawings. Other features, objects and advantages of the present disclosure will become more apparent from the description, drawings, and claims.

## BRIEF DESCRIPTION OF THE DRAWINGS

In order to describe the technical solutions of the embodiments of the present disclosure more clearly, the accompanying drawings required for describing the embodiments or for describing the conventional art will be briefly introduced as follows. Apparently, the accompanying drawings, in the following description, illustrate merely some embodiments of the present disclosure, and for a person of ordinary skill in the art, other drawings can also be obtained according to these accompanying drawings without making any creative efforts.

    FIG. 1 is a schematic flow chart of a method for identifying a prompt-$\gamma$ event in an embodiment.
    FIG. 2 is a schematic diagram of photon annihilation in a detector.
    FIG. 3 is a schematic flow chart of a method for identifying a prompt-$\gamma$ event in another embodiment.
    FIG. 4 is a schematic flow chart of a method for identifying a prompt-$\gamma$ event in an embodiment.
    FIG. 5 is a schematic flow chart of a PET imaging method in an embodiment.
    FIG. 6 is a schematic diagram of a transient gamma photon event identification device in another embodiment.
    FIG. 7 is a schematic diagram of a PET imaging device in an embodiment.
    FIG. 8 is a schematic diagram of an electronic computer device in an embodiment.

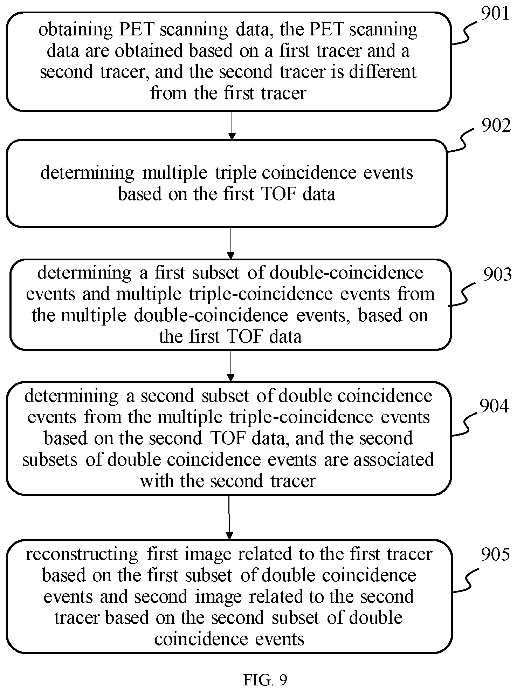

    FIG. 9 is a schematic flow chart of an iterative image decomposition method in another embodiment.

## DETAILED DESCRIPTION OF THE EMBODIMENTS

In order to make the objectives, technical solutions and advantages of the present disclosure more clearly understood, the present disclosure will be further described in detail with the accompanying drawings and embodiments. It should be understood that the specific embodiments described herein are only used to explain the present disclosure and not to limit the present disclosure.

It should be understood that when used in the description of the present disclosure and in the attached claims, the term "includes" indicates the presence of a described feature, whole, step, operation, element and/or component, but does not exclude the presence or addition of one or more other features, whole, step, operation, element, component and/or their sets.

In the description of this application and the attached claims, the terms "first", "second", "third", etc. are used only to distinguish the description and should not be construed as indicating or suggesting relative importance.

Reference to "one embodiment" or "some embodiments" as described in this application specification implies that specific features, structures or characteristics described in combination with those embodiments are included in one or more embodiments of this application. Thus, statements such as "in one embodiment", "in some embodiments", "in some other embodiments", "in some other embodiments" that appear in the differences of this specification do not necessarily refer to the same embodiments, but mean "one or more but not all embodiments", unless otherwise particularly emphasized. The terms "include", "contain", "have" and their variations mean "include but are not limited to", unless otherwise particularly emphasized.

Conventional PET single-scan imaging can only perform functional imaging of one tracer and has limited characterizing ability for complex life activities. Multiple PET scans can provide more comprehensive information about life activities, but they are costly in terms of economy and time, and registration problems between multiple scans can also affect the final imaging quality. Therefore, single PET scan imaging with multiple tracers is of great significance in clinical applications.

However, some nuclides, while decaying, emit transient gamma photon rays with a certain probability, which can produce triple coincidence events after positron annihilation. However, in traditional multi-tracer single PET scan imaging, only double coincidence events can be identified and output, and transient gamma photon events cannot be identified in triple coincidence events, which leads to the inability to effectively separate the double nuclide signals.

In view of this, the present disclosure presents a method for identifying transient gamma photon events, which can obtain TOF data of triple event using the time-of-flight situation of the double event and effectively identify transient gamma photon events in triple event using the TOF situation of the triple event, contributing to the separation of the double nuclide signal.

For the sake of understanding, some of the technical terms mentioned in the present disclosure are explained first.

Photon pair: a pair of photons flying in opposite directions, which can refer to a $\beta^+$ photon pair.

Coincidence event: The associated events that occur detected by coincidence detection technology are recorded as a coincidence count. Double coincidence events are two associated events that occur simultaneously. In the embodiments of the present disclosure, a triple coincidence event may refer to a double coincidence event produced by a photon pair and a transient gamma photon event produced by decay simultaneously due to the emission of transient gamma photon rays.

A prompt-$\gamma$ event, also known as a prompt-$\gamma$ event, refers to the occurrence of annihilation where, in addition to a pair of photons traveling in opposite directions, a single gamma photon is produced. Since the gamma photon occurs only on the order of picoseconds (ps) from the photon pair, the gamma photon is called a prompt-$\gamma$.

TOF: Time of Flight, flight time, in this application, refers to the difference in time when a pair of photons reach the PET detector, that is, the difference in detection time.

Detection position refers to the position at which the detector receives the photon.

Decay position refers to the position where decay occurs.

To illustrate the technical solution of the present disclosure, specific embodiments are presented below.

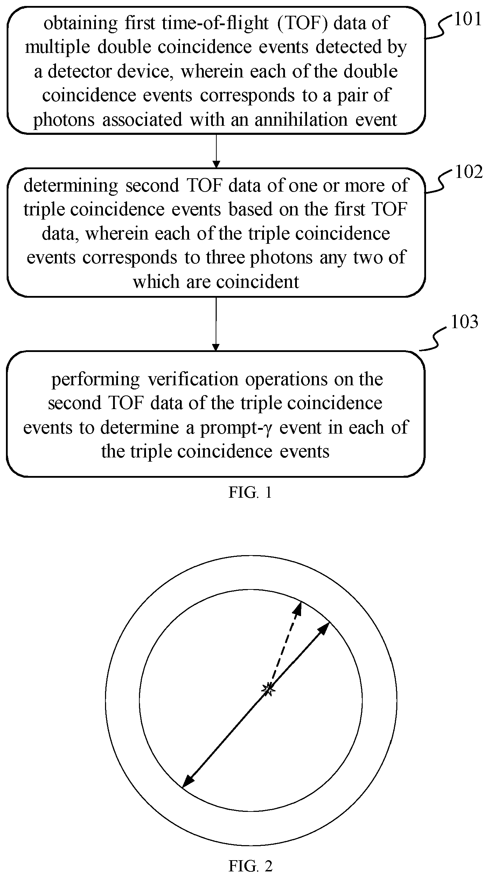

Please refer to FIG. 1, which shows a schematic diagram of the implementation process of a method for identifying prompt-$\gamma$ events provided by an embodiment of the present disclosure, which can be applied to an electronic device. The electronic device may be a computer, a PET device, or any other intelligent device capable of processing TOF data, for which the present disclosure is not restricted.

Specifically, the method for identifying the aforementioned transient gamma photon event may include steps S101 to S104.

Step S101: obtaining first time-of-flight (TOF) data of multiple double coincidence events detected by a detector device, wherein each of the double coincidence events corresponds to a pair of photons associated with an annihilation event.

In the embodiments of the present disclosure, the detector may refer to a PET detector. A PET detector may be a ring-shaped structure formed by multiple detector units for capturing photons produced when annihilation occurs. Electronic devices can obtain the first TOF data collected by the PET detector over a period of time through wired or wireless communication with the PET detector.

A double-coincidence event refers to a pair of coincidence events generated after the annihilation of a photon. When two single events are associated events that occur simultaneously, it indicates that the time difference (TOF) between the two single events is within the predetermined time window, that is, the time interval between the two single events is less than the preset threshold. The first TOF data for a double-coincidence event is generated based on the detector's detection of the double-coincidence event, carrying the flight time between the two double-coincidence events. The first TOF data can include the detection position and TOF information of the double coincidence event and can be represented in the form of list-mode data. Specifically, the first TOF data can be represented as $(p_1, p_2, t_{12})$. Wherein $p_1$ and $p_2$ are the detection positions of the photon. $t_{12}$ is the difference between the detection time of $p_1$, and $p_2$, that is, the flight time between the event $p_1$ and event $p_2$.

In the embodiments of the present disclosure, please refer to FIG. 2, since some nuclides emit transient gamma photon rays with a certain probability while decaying (as shown by the dotted line in FIG. 2), the double coincidence events detected by the detector may include double coincidence events (or true double coincidence events) produced by photon pairs, It may also include a double match event (or false double match event) consisting of a transient gamma photon event and an event corresponding to one of the photons in the photon pair.

Step S102, determining second TOF data of one or more of triple coincidence events based on the first TOF data, wherein each of the triple coincidence events corresponds to three photons, any two of which are coincident.

In the embodiments of the present disclosure, triple coincidence events are three concurrent associated events, which may refer to the double coincidence event produced by the photon pair and the transient gamma photon event produced by the simultaneous emission of the transient gamma photon ray during decay, and the flight times of these three events are temporally correlated. The second TOF data of the triplet event carries the TOF information between each pair of the triplet events and may include the detection positions of the three events of the triplet event, as well as the flight times between each pair of the three events.

Specifically, a triplet event has three photons, so the second TOF data can be expressed as:

$$\{(p_1, p_2, t_{12}), (p_1, p_3, t_{13}), (p_2, p_3, t_{23})\}。$$

Here, $p_1$, $p_2$, $p_3$ respectively represent the detection positions of the three photons, corresponding to the true double match event produced by the photon pair, and one transient gamma photon event. $t_{12}$ is the difference from the detection time of $p_1$ and $p_2$, $t_{13}$ is the difference from the detection time of $p_1$ and $p_3$, $t_{23}$ is the difference from the detection time of $p_2$ and $p_3$. According to the definition of flight time, we can get $t_{23}=t_{13}-t_{12}$. Based on this time-of-flight correlation, the second TOF data for the triple coincidence events can be determined according to the first TOF data.

Step S103: performing verification operations on the second TOF data of the triple coincidence events to determine a prompt-$\gamma$ event in each of the triple coincidence events.

In the embodiment of the present disclosure, after obtaining the second TOF data, one or more verification operations can be performed to determine the position of the transient gamma photon based on the detection positions of the three photons and the TOF information between each pair. The position of the transient gamma photon refers to the detection position of the transient gamma photon.

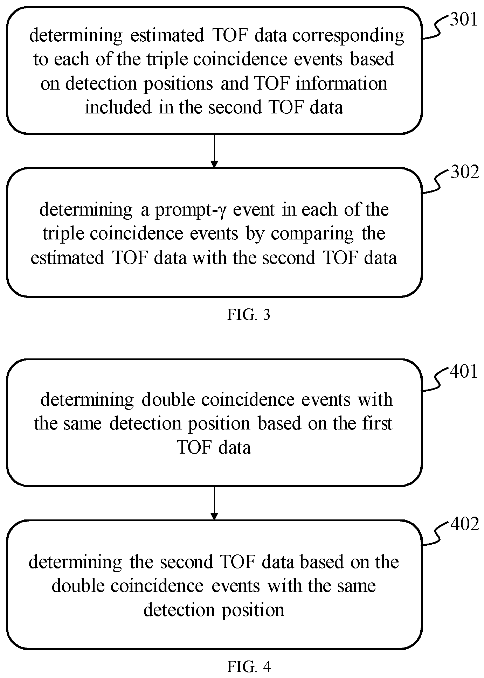

In embodiments of the present disclosure, as shown in FIG. 3, the verification operation may include steps S301 to S302.

Step S301, determining estimated TOF data corresponding to each of the triple coincidence events based on detection positions and TOF information included in the second TOF data.

In the embodiments of the present disclosure, a true double coincidence event refers to a coincidence event in which the two photons detected by the matching originated from the same annihilation event and neither of the two photons had any interaction with the medium before reaching the detector. One of the three detection positions of the triplet event must correspond to the transient gamma photon, and the other two correspond to the photon pairs produced by the electron-positron annihilation with an angle of $\pi$. For the second TOF data $\{(p_1, p_2, t_{12}), (p_1,p_3,t_{13}),(p_2,p_3,t_{23})\}$, it is known to be the TOF data of the triple-coincidence event, but it is unknown which of $p_1$, $p_2$ and $p_3$, and corresponds to the transient gamma photon event. Therefore, one of the triple coincidence events can be selected as the transient gamma photon event, with the corresponding detection position being the candidate transient gamma photon position; At this point, the remaining two events are selected as candidate true double coincidence events.

For example, if $p_1$ is selected as the candidate transient gamma photon position, then, $p_2$ or $p_3$ corresponds to one candidate true double coincidence event. If $p_2$ is selected as the candidate transient gamma photon position, then, $p_1$ or $p_3$ corresponds to one candidate true double coincidence event. If $p_3$ is selected as the candidate transient gamma photon position, then, $p_1$ or $p_2$ corresponds to one candidate true double coincidence event.

Step S302, determining a prompt-$\gamma$ event in each of the triple coincidence events by comparing the estimated TOF data with the second TOF data.

Determining the estimated TOF data includes: selecting candidate true double coincidence events and a candidate prompt-$\gamma$ photon positions based on each of the triple coincidence events; determining a candidate decay position based on the detection position and TOF information of the candidate true double coincidence events; determining an estimated TOF data of each group of a corresponding triple coincidence events based on the candidate decay position, the detection position of the candidate true double coincidence events, and the a detection position of the candidate prompt-$\gamma$ photon position.

Based on the detection location and TOF information of the candidate true double match event, the decay occurrence location corresponding to the candidate true double match event can be determined as the candidate decay location.

Specifically, Step S302 may include: determining one or more weights based on the detection position and TOF information of the candidate true double coincidence events; obtaining candidate decay positions by calculating a weighted sum of adding to the detection positions of the candidate true double coincidence events based on the weights.

Here, determining the weighting based on the detection position and TOF information of the candidate true double coincidence event may include: calculating the distance between the photon pairs corresponding to the candidate true double coincidence event based on the detection position of the candidate true double coincidence event, dividing the flight time corresponding to the TOF information by the distance between the photon pairs, and determining the weighting based on the division result and the speed of light.

We can take $p_1$, $p_2$ and $p_3$ respectively as the candidate positions of prompt gamma photons. Defined c as the speed of light, ":=" as the assignment symbol, and "||a - b||" as the distance between a and b.

Set $p_1$ as the position of the candidate transient gamma photon. At this point, the true double-coincidence event produced by the photon pair is $(p_2, p_3, t_{23})$, and the candidate decay position can be calculated by $p_{23} := \lambda p_2 + (1 - \lambda)p_3$, wherein the weighting weights $\lambda := (1 - c \cdot t_{23}/\|p_2 - p_3\|)/2$.

Set p2 as the position of the candidate transient gamma photon. At this point, the true double-coincidence event produced by the photon pair is $(p_1, p_3, t_{13})$, and the candidate decay position can be calculated by $p_{13} := \lambda p_1 + (1 - \lambda)p_3$, wherein the weighting weights $\lambda := (1 - c \cdot t_{13}/\|p_1 - p_3\|)/2$.

Set p3 as the position of the candidate transient gamma photon. At this point, the true double-coincidence event produced by the photon pair is $(p_2, p_3, t_{23})$, and the candidate decay position can be calculated by $p_{12} := \lambda p_1 + (1 - \lambda)p_2$ wherein the weighting weights $\lambda := (1 - c \cdot t_{12}/\|p_1 - p_2\|)/2$.

Here, the estimated TOF data can include TOF information between every two events in the triple coincidence event, which can be expressed as $(t'_{12}, t'_{13}, t'_{23})$. Specifically, for candidate decay positions, substituting the second TOF data $\{(p_1, p_2, t_{12}), (p_1, p_3, t_{13}), (p_2, p_3, t_{23})\}$ generates the corresponding estimated TOF data $(t'_{12}, t'_{13}, t'_{23})$.

In the embodiments of the present disclosure, determining the prompt-γ event in each of the triple coincidence events by comparing the estimated TOF data with the second TOF data includes: comparing the estimated TOF data with the second TOF data, in response to the estimated TOF data matching the second TOF data, determining the candidate prompt-γ photon position as the true prompt-γ photon; in response to the estimated TOF data not matching the second TOF data, reselecting another candidate true double coincidence event and another candidate prompt-γ photon.

If the estimated TOF data matches the second TOF data, it indicates that the candidate true double coincidence events and candidate transient gamma photon positions selected for the estimated TOF data are in line with the actual detection results, and thus the candidate transient gamma photon positions can be determined as transient gamma photon positions. If the estimated TOF data does not match the second TOF data, it indicates that the candidate true double coincidence events and candidate transient gamma photon positions selected for the estimated TOF data do not match the actual detection results, then this assumption of candidate true double coincidence events and candidate transient gamma photon positions should be discarded.

Specifically, in the verification process, the identification method provided in the present disclosure may also include: the estimated TOF data matching the second TOF data further includes: determining the TOF difference between the estimated TOF data and the second TOF data; confirming that the estimated TOF data matches the second TOF data if the TOF difference is less than the difference threshold.

In the embodiments of the present disclosure, the smaller the TOF difference, the closer the candidate decay position is to the decay position corresponding to the true double-coincidence event. If the TOF difference is less than the difference threshold, it can be confirmed that the estimated TOF data matches the second TOF data. If the TOF difference is greater than or equal to the error threshold, it can be confirmed that the estimated TOF data does not match the second TOF data.

Specifically, based on the second TOF data, the reference TOF vector can be determined, and the distance between the TOF vector corresponding to the estimated TOF data and the reference TOF vector can be calculated to obtain the TOF difference.

Here, the reference TOF vector characterizes the true value of the TOF vector of the triple - coincidence event. The reference TOF vector $(t_{12}, t_{13}, t_{23})$ can be determined based on the second TOF data $\{(p_1, p_2, t_{12}), (p_1, p_3, t_{13}), (p_2, p_3, t_{23})\}$. Based on the estimated TOF data, the corresponding TOF vector $(t'_{12}, t'_{13}, t'_{23})$ can be obtained. The TOF difference can be obtained by calculating the distance

$$\text{dist}\left((t_{12}, t_{13}, t_{23}), (t'_{12}, t'_{13}, t'_{23})\right),$$ between the TOF vector corresponding to the estimated TOF data and the reference TOF vector.

It is understandable that the distance is positively correlated with the TOF difference.

In the embodiments of the present disclosure, if p1, p2 and p3 are respectively regarded as transient gamma photon events, a combination of three candidate true double-coincidence events and candidate transient gamma photon positions can be obtained. The present disclosure can verify each of the combinations; It is also possible to verify each combination individually until the position of the transient gamma photon is determined, in which case the position of the transient gamma photon can be determined by at least one verification.

For example, let triple coincidence event data satisfy $p_1 := (1,0)$, $p_2 := (0,1)$, $p_3 := (0,0)$, reference TOF vector $(t_{12}, t_{13}, t_{23}) := (0,0,0)$. Then by classification discussion we have:

Suppose $p_1$ corresponds to the prompt gamma photon event, at this time the candidate decay position $p_{23} :=$ (0,0.5). The corresponding TOF vector is

$$\left(t'_{12}, t'_{13}, t'_{23}\right) = \left((\sqrt{5} - 1)/2, (\sqrt{5} - 1)/2, 0\right)$$

, which does not match the reference TOF vector.

Suppose $p_2$ corresponds to the prompt gamma photon event, at this time the candidate decay position $p_{13} := (0.5,0)$. The corresponding TOF vector is

$$\left(t'_{12}, t'_{13}, t'_{23}\right) = \left((1 - \sqrt{5})/2, 0, (\sqrt{5} - 1)/2\right)$$

, which does not match the reference TOF vector.

Suppose $p_3$ corresponds to the prompt gamma photon event, at this time the candidate decay position $p_{12} := (0.5,0.5)$. The corresponding TOF vector is

$$\left(t'_{12}, t'_{13}, t'_{23}\right) = (0,0,0)$$ , the distance from the reference TOF vector is 0 (that is, match the reference TOF vector).

So it can be judged $p_{12}$ as decay position of a true double match event; $p_3$ is the position of the transient gamma photon.

In the embodiments of the present disclosure, an event detected at the position of a transient gamma photon in a triple coincidence event is a transient gamma photon event.

In the embodiments of the present disclosure, by obtaining the first TOF data of the double-coincidence event detected by the detector, the second TOF data of the triple- coincidence event is determined based on the first TOF data, and one or more verification operations are performed to determine the position of the transient gamma photon, and based on the position of the transient gamma photon, the transient gamma photon event is determined in the triple-conforming event, The TOF data of the triple event can be obtained using the TOF situation of the double event, and the position of the transient gamma photon can be verified using the TOF situation of the triple event to effectively identify the transient gamma photon event in the triple event, which helps to separate the double nuclide signal.

As shown in FIG. 4, in some embodiments of the present disclosure, determining second TOF data of one or more triple coincidence events based on the first TOF data may include steps S401 to S402.

Step S401, determining double coincidence events with the same detection position based on the first TOF data.

It is understandable that if there is an event corresponding to the same photon in two different double-coincidence events, then the detection positions of these two events should be the same. For triple-coincidence events, there must be an event corresponding to the same photon between the two double-coincidence events. Based on this principle, it can be determined that there are double coincidence events with the same detection position in the first TOF data. For example, the first TOF data($p_1$, $p_2$, $t_{12}$) and the first TOF data($p_1$, $p_3$, $t_{13}$) represent two different double-coincidence events with the same detection position, indicating that these two double-coincidence events are double-coincidence events with the same detection position and that there is an event corresponding to the same photon between these two double-coincidence events (i.e., the $p_1$ corresponding event).

By comparing the detection positions pairwise of the first TOF data for all double-coincidence events, at least one pair of double-coincidence events with the same detection position can be obtained.

Step S402: determining the second TOF data based on the double coincidence events with the same detection position.

In the embodiments of the present disclosure, for a pair of double-coincidence events with the same detection position, the same detection position can correspond to one event, and the two different detection positions between them can correspond to one event respectively, thereby determining the second TOF data for the triple coincidence events. For example, the probing positions $p_1$ between the first TOF data($p_1$, $p_2$, $t_{12}$) and the first TOF data($p_1$, $p_3$, $t_{13}$) are the same and $p_1$ can correspond to one event, then $p_2$, $p_3$ can correspond to an event respectively.

Specifically, the present disclosure provides two ways to obtain the second TOF data:

Method 1:

The aforementioned events with the same detection position include three double-coincidence events, in which there is an identical detection position between every two double-coincidence events in the three double-coincidence events, and the identical detection positions between every two double-coincidence events are different.

For example, ($p_1$,$p_2$,$t_{12}$), ($p_1$,$p_3$,$t_{13}$), ($p_2$,$p_3$,$t_{23}$) represent the three double coincidence events mentioned above. The same detection position between ($p_1$,$p_2$,$t_{12}$) and ($p_1$, $p_3$,$t_{13}$) is $p_1$ , the same detection position between ($p_1$, $p_2$, $t_{12}$), ($p_2$, $p_3$,$t_{23}$) is $p_2$, the same detection position between ($p_1$, $p_3$,$t_{13}$), ($p_2$,$p_3$,$t_{23}$) is $p_3$.

At this point, the second TOF data is determined based on the existence of double coincidence events with the same detection position, which may include: checking whether TOF information of the three double coincidence events satisfies the time condition; generating second TOF data based on the detection position and TOF information of the three double coincidence events if the TOF information of the three double meets the time condition.

As described earlier, the time condition that the TOF information of the three coincidence events should satisfy can be expressed as: $t_{23} = t_{13} - t_{12}$

If the TOF information of the three double-coincidence events meets this time condition, it indicates that these three double-coincidence events are the true double-coincidence events produced by the photon pairs and the transient gamma photon events, then the second TOF data $\{(p_1, p_2, t_{12}), (p_1,p_3,t_{13}),(p_2, p_3,t_{23})\}$ can be generated based on the detection position $p_1, p_2, p_3$, and TOF information $t_{12},t_{13},t_{23}$ contained in the three double-coincidence events.

If the TOF information of the three double coincidence events does not meet the time condition, it indicates that the three double coincidence events are not true double coincidence events produced by photon pairs and transient gamma photon events produced by decay along with the emission of transient gamma photon rays. In this case, the three double coincidence events can be skipped.

Method 1 can filter out double coincidence events that have the same detection position and meet the time condition from the first TOF data collected, and then generate the second TOF data of the triple coincidence events, which can ensure the accuracy of the second TOF data of the triple coincidence events.

Method 2:

The events with the same detection position mentioned above include two double-coincidence events. For example, the two double coincidence events could be $\{(p_1, p_2,t_{12}), (p_1,p_3,t_{13})\}$, $\{(p_2,p_3,t_{23}),(p_1,p_3,t_{13})\}$, or, $\{(p_2,p_3,t_{23}), (p_2, p_3,t_{23})\}$.

At this point, the determination of the second TOF data may include: determining the TOF information of the third double coincidence event based on the TOF information of the two double coincidence events; generating the second TOF data based on the detection position and TOF information of the two double coincidence events, as well as the TOF information of the third double coincidence event.

Specifically, according to the time relationshipt$_{23}$ = $t_{13}$ - $t_{12}$, the TOF information of the third double match event can be determined. At this point, the probing positions $p_1,p_2,p_3$ of the two double coincidence events can contain probing positions, and the TOF information between the two double coincidence events, combined with the TOF information of the third double-coincidence event, a second TOF data $\{(p_1, p_2,t_{12}), (p_1,p_3,t_{13}),(p_2, p_3,t_{23})\}$ can be generated.

In the case of the two double coincidence events$\{(p_1,p_2,t_{12}), (p_1,p_3)t_{13})\}$, the third double coincidence event can be computed by $(p_2, p_3,t_{23} := t_{13} - t_{23})$.

This approach can supplement the TOF information of the third double match event when it is missing, thereby generating the second TOF data, which can improve the universality of the algorithm.

In some embodiments of the present disclosure, after obtaining the transient gamma photon event, it may also include: eliminating the transient gamma photon event from the tripartite event to obtain the true double compliance event. Alternatively, the TOF data corresponding to the transient gamma photon event is removed from the second TOF data. For example, after determining that $p_{12}$ as the target decay position, and $p_3$ is a transient gamma photon event, it can be determined that the true double match event is$(p_1,p_2,t_{12})$, deleted $(p_1, p_3,t_{13})$和 $(p_2,p_3,t_{23})$.

True double coincidence events can be used for dual-nuclide imaging, to determine image quality, or to assess detector performance, and there are no restrictions on the application scenarios for true double coincidence events in this application.

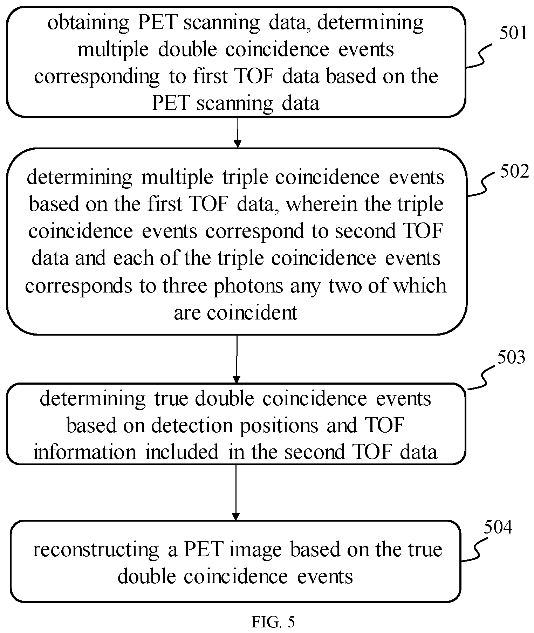

Please refer to FIG. 5, which shows a schematic diagram of the implementation process of a PET imaging method provided by an embodiment of the present disclosure, which can be applied to electronic devices. The electronic device may be a computer, a PET device, or any other intelligent device capable of imaging PET data, to which the present disclosure is not limited.

The PET imaging method may include: steps S501 to S504.
Step S501, obtaining PET scanning data; determining multiple double coincidence events corresponding to first TOF data based on the PET scanning data.

A double coincidence event is a pair of coincidence events produced after photon annihilation.

Step S502, determining multiple triple coincidence events based on the first TOF data, wherein the triple coincidence events correspond to second TOF data and each of the triple coincidence events corresponds to three photons, any two of which are coincident; Among them, a triple-coincidence event is an event that can be pairwise matched after photon annihilation.

Step S503: determining true double coincidence events based on detection positions and TOF information included in the second TOF data.
Among them, the determining true double coincidence events based on the position of each single event in each set of triple coincidence events and the corresponding set of second TOF data, including: selecting candidate true double coincidence events and candidate prompt-γ photon positions based on the triple coincidence events; obtaining candidate decay position based on the detection position and TOF information of the candidate true double coincidence events; obtaining estimated TOF data of each group of triple coincidence events based on the candidate decay position, the detection position of the candidate true double coincidence events, and the candidate prompt-γ photon position; determining the candidate prompt-γ photon position as the prompt-γ photon position in response to the estimated TOF data matching the second TOF data.

The specific implementation of steps S501 to S503 is illustrated in FIGS. 1 to 4, with the difference being: FIGS. 1-4 identify the candidate transient gamma photon position as the transient gamma photon position, while FIG. 5 identifies the candidate true double coincidence event as the true double coincidence event, that is, FIG. 5 focuses on identifying the true double coincidence event among the triple coincidence events, which is not elaborated in this application.

Step S504: reconstructing a PET image based on the true double coincidence events.

Among them, the above-mentioned TOF data can be TOF-PET data, and correspondingly, the TOF image can be a TOF-PET image. Based on the TOF data corresponding to the true double match event, image reconstruction can be carried out using the existing methods to obtain the TOF image.

In the embodiments of the present disclosure, the TOF situation of the triple - coincidence event can be used to determine the true double-match event, facilitating the reconstruction of the TOF data corresponding to the true double-match event to obtain the TOF image, which helps to improve the reliability of the TOF image.

It should be noted that for the embodiments of the aforementioned methods, for the sake of simplicity, they are all expressed as a series of combinations of actions, but those skilled in the art should be aware that the present disclosure is not limited by the sequence of actions described, because according to the present disclosure, certain steps can be carried out in a different sequence.

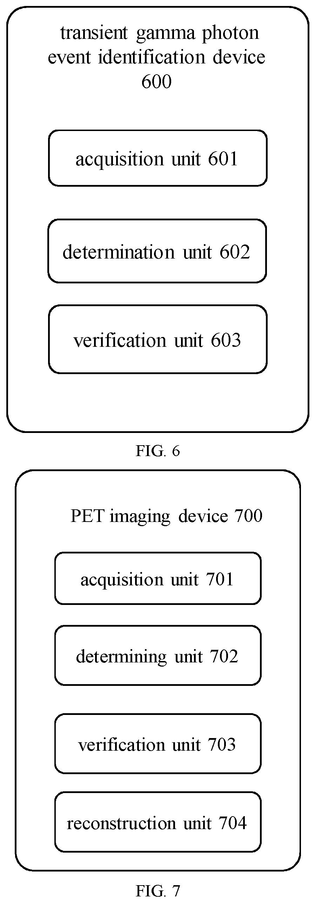

As shown in FIG. 6, it is a schematic diagram of the structure of a transient gamma photon event recognition device 600 provided by the embodiments of the present disclosure, which is configured on an electronic device.

Specifically, the transient gamma photon event identification device 600 may include: acquisition unit 601, configured to obtain first time-of-flight (TOF) data of multiple double coincidence events detected by a detector device, wherein each of the double coincidence events corresponds to a pair of photons associated with an annihilation event; and determination unit 602, configured to determine second TOF data of one or more triple coincidence events based on the first TOF data, wherein each of the triple coincidence events corresponds to three photons any two of which are coincident; and verification unit 603, configured to perform verification operations on the second TOF data of the triple coincidence events to determine a prompt-γ event in each of the triple coincidence events.

Among them, verification unit 603 is specifically configured to: determine estimated TOF data corresponding to each of the triple coincidence events based on detection positions and TOF information included in the second TOF data; and determine a prompt-γ event in each of the triple coincidence events by comparing the estimated TOF data with the second TOF data.

In some embodiments of the present disclosure, verification unit 603 may be specifically configured to select candidate true double coincidence event and a candidate prompt-γ photon based on each of the triple coincidence events; and determine a candidate decay position based on the detection position and TOF information of the candidate true double coincidence event; and determine an estimated TOF data of a corresponding triple coincidence event based on the candidate decay position, the detection position of the candidate true double coincidence event, and a detection position of the candidate prompt-γ photon.

In some embodiments of the present disclosure, the verification unit 603 may be specifically configured to determine weights based on the detection position and TOF information of the candidate true double coincidence events; and to obtain candidate decay positions by adding to the detection positions of the candidate true double coincidence events based on the weights.

It should be noted that for the convenience and conciseness of description, the specific working process of the identification device 600 of the transient gamma photon event mentioned above can be referred to the corresponding process of the methods described in FIGS. 1 to 4, and will not be elaborated here.

As shown in FIG. 7, it is a schematic diagram of the structure of a PET imaging device 700 provided in an embodiment of the present disclosure, which is configured on an electronic device.

Specifically, the PET imaging device 700 includes:

an acquisition unit 701, configured to obtain PET scanning data; and determine multiple double coincidence events corresponding to first TOF data based on the PET scanning data; determining unit 702, configured to determine multiple triple coincidence events based on the first TOF data, wherein the triple coincidence events correspond to second TOF data and each of the triple coincidence events corresponds to three photons any two of which are coincident;

a verification unit 703, configured to determine true double coincidence events based on detection positions and TOF information included in the second TOF data;

a reconstruction unit 704, configured to reconstruct a PET image based on the true double coincidence events.

The verification unit 703 is specifically configured to determine estimated TOF data corresponding to each of the triple coincidence events based on detection positions and TOF information included in the second TOF data; and determine a prompt-γ event in each of the triple coincidence events by comparing the estimated TOF data with the second TOF data. It should be noted that for the convenience and conciseness of description, the specific working process of the PET data reconstruction device 700 mentioned above can be referred to the corresponding process of the method described in FIG. 5 and will not be elaborated here.

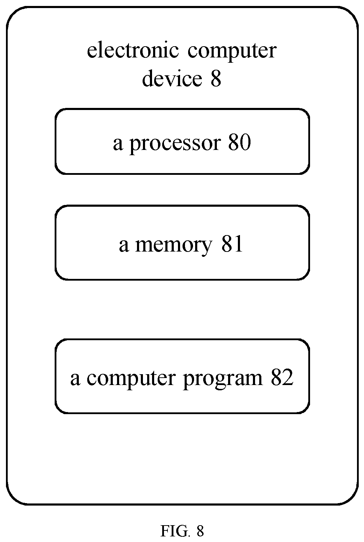

As shown in FIG. 8, it is a schematic diagram of an electronic computer device 8 provided in an embodiment of the present disclosure. Specifically, the electronic computer device 8 may include: a processor 80, a memory 81 and a computer program 82 stored in the memory 81 and capable of running on the processor 80. When the processor 80 executes the computer program 82, it performs the following steps:

Step 901, obtaining PET scanning data, the PET scanning data are obtained based on a first tracer and a second tracer, and the second tracer is different from the first tracer;

Step 902, determining multiple triple coincidence events based on the first TOF data, wherein the triple coincidence events correspond to second TOF data and each of the triple coincidence events corresponds to three photons any two of which are coincident;

Step 903, determining a first subset of double-coincidence events and multiple triple-coincidence events from the multiple double-coincidence events, based on the first TOF data; wherein triple-coincidence events correspond to second TOF data, and the first subset of double-coincidence events is associated with the first tracer;

Step 904, determining a second subset of double coincidence events from the multiple triple-coincidence events based on the second TOF data, and the second subsets of double coincidence events are associated with the second tracer;

Step 905, reconstructing first image related to the first tracer based on the first subset of double coincidence events and second image related to the second tracer based on the second subset of double coincidence events.

Among them, the second tracer is different from the first tracer. Specifically, one of the first tracer and the second tracer includes $\beta^+$ particle decay, and the other one includes $\beta^+\gamma$ decay. For example, the first tracer only undergoes $\beta^+$ decay. The first tracer includes, for example, F18, C11. The second tracer can occur $\beta^+\gamma$ decay (that is, $\beta^+$ decay plus a transient photon). For example, the second tracer includes I124, Ga68. Of course, they can also change each other, the first tracer can occur $\beta^+\gamma$ decay; the second tracer can occur $\beta^+$ decay.

Among them, if there is a single event with the same detection position and detection time in both sets of double coincidence events, then the three distinct single events in the two sets of double coincidence events can constitute a set of triple coincidence events.

Here, the method of determining the transient gamma photon event based on the position of the transient gamma photon can be described in FIGS. 1-4, which will not be elaborated in this application. Electronic Device 8, when processing TOF data, can delete the TOF data corresponding to the transient gamma photon event from the second TOF data, thereby deleting the non-true double compliance data.

In this way, the remaining data in the second TOF can represent the data of the true double match event, facilitating data analysis and PET data reconstruction.

The computer program may be split into one or more modules/units which are stored in the memory 81 and executed by the processor 80 to complete the present disclosure. One or more of the modules/units may be a series of computer program instruction segments capable of performing a specific function, which are used to describe the execution process of the computer program in the electronic device 8.

The electronic device 8 may include, but not be limited to, a processor 80 and a memory 81. It can be understood by those skilled in the art that FIG. 8 is merely an example of the electronic device 8 and does not constitute a limitation on the electronic device 8, which may include more or fewer components than shown, or combine certain components, or different components, for example, the electronic device 8 May also include input/output devices, network access devices, buses, etc.

The Processor 80 can be a Central Processing Unit (CPU), or other general-purpose processors, or Digital Signal processors. DSP, Application Specific Integrated Circuit (ASIC), off-the-shelf programmable gate arrays or other programmable logic devices, discrete gates or transistor logic devices, discrete hardware components, etc. General-purpose processors can be microprocessors or that processor or any conventional processor, etc.

The memory 81 May be an internal storage unit of the electronic device 8, such as the hard disk or memory of the electronic device 8. The memory 81 May also be an external storage device of the electronic device 8, such as a plug-in hard disk, Smart Media Card (SMC), Secure Digital (SD) Card, Flash Card, etc. equipped on the electronic device 8. Further, the memory 81 May include both an internal storage unit of the electronic device 8 and an external storage device. Memory 81 is used to store the computer program as well as other programs and data required by the electronic device 8. The memory 81 May also be used to temporarily store data that has been or will be output.

It should be noted that for the convenience and conciseness of the description, the structure of the electronic device 8 mentioned above may also refer to the specific description of the structure in the method embodiments and will not be elaborated here.

It can be clearly understood by those skilled in the art that for the sake of convenience and conciseness of description, only the division of the above-mentioned functional units and modules is illustrated as an example. In practical application, the above-mentioned functional allocation can be accomplished by different functional units and modules as needed, that is, the internal structure of the device is divided into different functional units or modules. To complete all or part of the functions described above. The functional units and modules in the embodiments may be integrated into one processing unit, or the units may exist physically separately, or two or more units may be integrated into one unit, and the integrated units may be implemented either in the form of hardware or in the form of software functional units. In addition, the specific names of the functional units and modules are only for the purpose of distinguishing them from each other and are not intended to limit the scope of protection of the present disclosure. The specific working processes of the units and modules in the aforementioned system may refer to the corresponding processes in the embodiments of the aforementioned method and will not be elaborated here.

In the above embodiments, the descriptions of each embodiment have their own emphasis. For parts not detailed or recorded in one embodiment, refer to the relevant descriptions of other embodiments.

It can be perceived by those skilled in the art that, in combination with the units and algorithmic steps of the examples described in the embodiments disclosed herein, they can be implemented using electronic hardware, or a combination of computer software and electronic hardware. Whether these functions are performed in a hardware or software manner depends on the specific application and design constraints of the technical solution. A professional technician may use different methods for each specific application to implement the described functions, but such implementation should not be considered beyond the scope of the present disclosure.

In the embodiments provided in the present disclosure, it should be understood that the disclosed devices/equipment and methods can be implemented by other means. For example, the device/device embodiments described above are merely illustrative. For example, the division of the said module or unit is merely a logical functional division. In actual implementation, there can be other divisions, such as multiple units or components can be combined or integrated into another system, or some features can be ignored or not executed. Another point is that the coupling or direct coupling or communication connection between each other shown or discussed can be through some interfaces, and the indirect coupling or communication connection of the device or unit can be electrical, mechanical or otherwise.

The units described as separate components may or may not be physically separate, and the components shown as units may or may not be physical units, that is, they may be located in one place or distributed over multiple network units. Some or all of the units may be selected as needed to achieve the purpose of this embodiment.

In addition, the functional units in each embodiment of the present disclosure may be integrated into one processing unit, or the units may exist physically separately, or two or more units may be integrated into one unit. The integrated units may be implemented either in the form of hardware or in the form of software functional units.

When the integrated module/unit is implemented in the form of a software functional unit and sold or used as an independent product, it can be stored in a computer-readable storage medium. Based on this understanding, the present disclosure implements all or part of the processes of the embodiments described above, which can also be accomplished by instructs the relevant hardware through a computer program that is stored in a computer-readable storage medium, which, when executed by the processor, can implement the steps of each embodiments described above. Wherein the computer program includes computer program code, which can be in the form of source code, object code, executable file or some intermediate form, etc. The computer-readable medium may include: Any entity or device capable of carrying the computer program code, recording medium, USB flash drive, portable hard disk, floppy disk, optical disc, computer Memory, Read-Only Memory (ROM), Random Access memory (Random Access Memory) RAM, electrical carrier signals, telecommunication signals, and software distribution media, etc. It should be noted that the content included in the computer-readable medium may be appropriately increased or decreased in accordance with the requirements of legislation and patent practice in the jurisdiction. For example, in some jurisdictions, computer-readable medium does not include electrical carrier signals and telecommunication signals in accordance with legislation and patent practice.

A person of ordinary skill in the art may understand that implementation of all or part of the processes in the methods of the above embodiments may be completed by instructing the relevant hardware through a computer program. The computer program may be stored in a non-transitory computer-readable storage medium. When the computer program is executed, it may include the processes of the respective methods according to the foregoing embodiments. Any reference to memory, database or other medium used of the embodiments provided in the present disclosure may include at least one of a non-transitory or a transitory memory. The non-transitory memory may include a read-only memory (ROM), a magnetic tape, a floppy disk, a flash memory, an optical memory, a high-density embedded non-transitory memory, a resistive random-access memory (ReRAM), a magneto resistive random-access memory (MRAM), a ferroelectric random-access memory (FRAM), a phase change memory (PCM), or a graphene memory, etc. The transitory memory may include a random-access memory (RAM) or an external cache memory, etc. As an illustration rather than a limitation, the random-access memory may be in various forms, such as a static random-access memory (SRAM) or a dynamic random-access memory (DRAM), etc. The databases involved in the embodiments provided by the present disclosure may include at least one of a relational databases and a non-relational database. The non-relational database may include, but is not limited to, a blockchain-based distributed database, etc. The processor involved in the embodiments provided by the present disclosure may be, but is not limited to, a general purpose processor, a central processor, a graphics processor, a digital signal processor, a programmable logic device, a data processing logic device based on quantum computation, and the like.

The technical features in the above embodiments may be combined arbitrarily. For concise description, not all possible combinations of the technical features in the above embodiments are described. However, provided that they do not conflict with each other, all combinations of the technical features are to be considered to be within the scope of protection of the present disclosure.

The above-mentioned embodiments only describe several implementations of the present disclosure, and their description is specific and detailed, but should not be understood as a limitation on the protection scope of the present disclosure. It should be noted that, for a person of ordinary skill in the art, various variations and improvements can be further made without departing from the conception of the present disclosure, and these all fall within the protection scope of the present disclosure. Therefore, the protection scope of the present disclosure should be subject to the appended claims.

**Claims**

1. A method for identifying a prompt-$\gamma$ event, comprising:

   obtaining first time-of-flight (TOF) data of multiple double coincidence events detected by a detector device, wherein each of the double coincidence events corresponds to a pair of photons associated with an annihilation event; determining second TOF data of one or more triple coincidence events based on the first TOF data, wherein each of the triple coincidence events corresponds to three photons, any two of which are coincident; performing verification operations on the second TOF data of the triple coincidence events to determine a prompt-$\gamma$ event in each of the triple coincidence events; wherein each of the verification operations includes:

   determining estimated TOF data corresponding to each of the triple coincidence events based on detection positions and TOF information included in the second TOF data; and determining the prompt-$\gamma$ event in each of the triple coincidence events by comparing the estimated TOF data with the second TOF data.

2. The method for identifying a prompt-$\gamma$ event of claim 1, wherein determining the estimated TOF data comprises:

   selecting candidate true double coincidence event and a candidate prompt-$\gamma$ photon based on each of the triple coincidence events; determining a candidate decay position based on the detection position and TOF information of the candidate true double coincidence event; determining an estimated TOF data of a corresponding triple coincidence event based on the candidate decay position, the detection position of the candidate true double coincidence event, and a detection position of the candidate prompt-$\gamma$ photon.

3. The method for identifying a prompt-$\gamma$ event of claim 2, wherein determining the prompt-$\gamma$ event in each of the triple coincidence events by comparing the estimated TOF data with the second TOF data comprises:

   comparing the estimated TOF data with the second TOF data, in response to the estimated TOF data matching the second TOF data, determining the candidate prompt-$\gamma$ photon as the true prompt-$\gamma$ photon; or in response to the estimated TOF data not matching the second TOF data, reselecting another candidate true double coincidence event and another candidate prompt-$\gamma$ photon.

4. The method for identifying a prompt-$\gamma$ event of claim 3, wherein the estimated TOF data matching the second TOF data further includes:

   determining a TOF difference between the estimated TOF data and the second TOF data; confirming that the estimated TOF data matches the second TOF data if the TOF difference is less than a difference threshold.

5. The method for identifying a prompt-$\gamma$ event of claim 4, wherein determining the TOF difference between the estimated TOF data and the second TOF data includes:

   determining a reference TOF vector based on the second TOF data; obtaining TOF deference by calculating the distance between the TOF vector corresponding to the estimated TOF data and the reference TOF vector.

6. The method for identifying a prompt-γ event of claim 2-5, wherein determining the candidate decay position based on the detection position and the TOF information of the candidate true double coincidence events, including:

determining one or more weights based on the detection position and TOF information of the candidate true double coincidence events; obtaining candidate decay positions by calculating a weighted sum of the detection positions of the candidate true double coincidence events based on the weights.

7. The method for identifying a prompt-γ event of any one of claims 1-6, wherein determining the second TOF data of one or more triple coincidence events based on the first TOF data, including:

determining double coincidence events with the same detection position based on the first TOF data; determining the second TOF data based on the double coincidence events with the same detection position.

8. The method for identifying a prompt-γ event of claim 7, wherein the double coincidence events with the same detection position include three double coincidence events, and any two of the three double coincidence events have a same detection position; determining the second TOF data based on the double coincidence events with the same detection position, including:

checking whether the TOF information of the three double coincidence events satisfies a time condition; generating second TOF data based on the detection position and TOF information of the three double coincidence events if the TOF information of the three double coincidence events satisfies the time condition.

9. The method for identifying a prompt-γ event of claim 7-8, wherein the double coincidence events with the same detection position include two double coincidence events; determining the second TOF data based on the double coincidence events with the same detection position, including:

determining TOF information of a third double coincidence event based on the TOF information of the two double coincidence events;

generating the second TOF data based on the detection position and TOF information of the two double coincidence events, and the TOF information of the third double coincidence event.

10. A positron emission tomography (PET) imaging method, comprising:

obtaining PET scanning data; determining multiple double coincidence events corresponding to first TOF data based on the PET scanning data; determining multiple triple coincidence events based on the first TOF data, wherein the triple coincidence events correspond to second TOF data and each of the triple coincidence events corresponds to three photons any two of which are coincident; determining true double coincidence events based on detection positions and TOF information included in the second TOF data; reconstructing a PET image based on the true double coincidence events.

11. The PET imaging method of claim 10, wherein the determining true double coincidence events based on detection positions and TOF information included in the second TOF data, including:

determining estimated TOF data corresponding to each of the triple coincidence events based on detection positions and TOF information included in the second TOF data; and determining a prompt-γ event in each of the triple coincidence events by comparing the estimated TOF data with the second TOF data.

12. The PET imaging method of claim 11, wherein the determining the estimated TOF data comprises:

selecting candidate true double coincidence event and a candidate prompt-γ photon based on each of the triple coincidence events; determining a candidate decay position based on the detection position and TOF information of the candidate true double coincidence event; determining an estimated TOF data of a corresponding triple coincidence event based on the candidate decay position, the detection position of the candidate true double coincidence event, and a detection position of the candidate prompt-γ photon.

13. The PET imaging method of claim 12, wherein the determining the prompt-γ event in each of the triple coincidence events by comparing the estimated TOF data with the second TOF data comprises:

comparing the estimated TOF data with the second TOF data,
in response to the estimated TOF data matching the second TOF data, determining the candidate prompt-γ photon as the true prompt-γ photon; or in response to the estimated TOF data not matching the second TOF data, reselecting another candidate true double coincidence event and another candidate prompt-γ photon.

14. An electronic computer device, comprising a memory and a processor, the memory storing a computer program, wherein the processor performs the following steps when executing the computer program:

obtaining PET scanning data, the PET scanning data are obtained based on a first tracer and a second tracer, and the second tracer is different from the first tracer;
determining multiple double coincidence events based on the PET scanning data, with each pair of double coincidence events corresponding to a first TOF data;
determining a first subset of double-coincidence events and multiple triple-coincidence events from the multiple double-coincidence events, based on the first TOF data; wherein triple-coincidence events correspond to second TOF data, and the first subset of double-coincidence events is associated with the first tracer;
determining a second subset of double coincidence events from the multiple triple-coincidence events based on the second TOF data, and the second subsets of double coincidence events are associated with the second tracer;
reconstructing first image related to the first tracer based on the first subset of double coincidence events and second image related to the second tracer based on the second subset of double coincidence events.

15. The electronic computer device of claim 19, wherein the second tracer is different from the first tracer, including: one of the first tracer and the second tracer including $\beta^+$ particle decay; the other one including $\beta^+\gamma$ decay.

obtaining first time-of-flight (TOF) data of multiple double coincidence events detected by a detector device, wherein each of the double coincidence events corresponds to a pair of photons associated with an annihilation event ⟋101

determining second TOF data of one or more of triple coincidence events based on the first TOF data, wherein each of the triple coincidence events corresponds to three photons any two of which are coincident ⟋102

⟋103

performing verification operations on the second TOF data of the triple coincidence events to determine a prompt-γ event in each of the triple coincidence events

FIG. 1

FIG. 2

301

determining estimated TOF data corresponding
to each of the triple coincidence events based
on detection positions and TOF information
included in the second TOF data

302

determining a prompt-γ event in each of the
triple coincidence events by comparing the
estimated TOF data with the second TOF data

FIG. 3

401

determining double coincidence events with
the same detection position based on the first
TOF data

402

determining the second TOF data based on the
double coincidence events with the same
detection position

FIG. 4

obtaining PET scanning data, determining multiple double coincidence events corresponding to first TOF data based on the PET scanning data

501

determining multiple triple coincidence events based on the first TOF data, wherein the triple coincidence events correspond to second TOF data and each of the triple coincidence events corresponds to three photons any two of which are coincident

502

determining true double coincidence events based on detection positions and TOF information included in the second TOF data

503

reconstructing a PET image based on the true double coincidence events

504

FIG. 5

transient gamma photon
event identification device
600

acquisition unit 601

determination unit 602

verification unit 603

FIG. 6

PET imaging device 700

acquisition unit 701

determining unit 702

verification unit 703

reconstruction unit 704

FIG. 7

electronic computer
device 8

a processor 80

a memory 81

a computer program 82

FIG. 8

obtaining PET scanning data, the PET scanning
data are obtained based on a first tracer and a
second tracer, and the second tracer is different
from the first tracer
901

determining multiple triple coincidence events
based on the first TOF data
902

determining a first subset of double-coincidence
events and multiple triple-coincidence events from
the multiple double-coincidence events, based on
the first TOF data
903

determining a second subset of double coincidence
events from the multiple triple-coincidence events
based on the second TOF data, and the second
subsets of double coincidence events are associated
with the second tracer
904

reconstructing first image related to the first tracer
based on the first subset of double coincidence
events and second image related to the second
tracer based on the second subset of double
coincidence events
905

FIG. 9

# EUROPEAN SEARCH REPORT

**Application Number**

EP 25 20 2120

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/185339 A1 (LAGE EDUARDO M [US] ET AL) 2 July 2015 (2015-07-02) * paragraphs [0039], [0040], [0056] - [0062], [0072] - [0076], [0078], [0079], [0081]; figures 1-5 * | 1-15 | INV. G01T1/29 A61B6/03 |
| X | HOFGARD ELYSSA F ET AL: "Simultaneous Multi-Isotope PET: A Computational Framework for Line of Response (LOR) Identification", 2020 IEEE NUCLEAR SCIENCE SYMPOSIUM AND MEDICAL IMAGING CONFERENCE (NSS/MIC), IEEE, 31 October 2020 (2020-10-31), pages 1-2, XP033958994, DOI: 10.1109/NSS/MIC42677.2020.9507891 [retrieved on 2021-08-04] * abstract; figure 1 * * page 1 - page 2 * | 1-3, 10-15 | |
| A | ANDREYEV A ET AL: "Feasibility study of dual isotope PET", NUCLEAR SCIENCE SYMPOSIUM CONFERENCE RECORD (NSS/MIC), 2010 IEEE, IEEE, 30 October 2010 (2010-10-30), pages 2108-2111, XP032054425, DOI: 10.1109/NSSMIC.2010.5874150 ISBN: 978-1-4244-9106-3 * abstract; figures 2,3,5 * * section B.; page 2109, column 2 - page 2110, column 1 * | 1,10,14 | **TECHNICAL FIELDS SEARCHED (IPC)** G01T A61B |

The present search report has been drawn up for all claims

| Place of search | Date of completion of the search | Examiner |
|---|---|---|
| The Hague | 14 January 2026 | Van Ouytsel, Krist'l |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 2120

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2026

| Patent document cited in search report | Publication date | Patent family member(s) | Publication date |
|---|---|---|---|
| US 2015185339 A1 | 02-07-2015 | US 2015185339 A1<br>WO 2013188011 A1 | 02-07-2015<br>19-12-2013 |

EPO FORM P0459

For more details about this annex : see Official Journal of the European Patent Office, No. 12/82